(19) Europäisches Patentamt
European Patent Office
Office européen des brevets

(11) Publication number: 0 295 567 B1

(12) EUROPEAN PATENT SPECIFICATION

(45) Date of publication of patent specification: 12.10.94  (51) Int. Cl.5: C12M 1/12

(21) Application number: 88109151.6

(22) Date of filing: 08.06.88

(54) Tubular bioreactor.

(30) Priority: 13.06.87 JP 146245/87

(43) Date of publication of application:
21.12.88 Bulletin 88/51

(45) Publication of the grant of the patent:
12.10.94 Bulletin 94/41

(84) Designated Contracting States:
CH DE FR GB LI NL SE

(56) References cited:
EP-A- 0 195 094
DE-A- 3 215 003
US-A- 3 969 190
US-A- 4 446 229

SOVIET INVENTIONS ILLUSTRATED, Section
Chemical, Week B49, 23 January 1980, no.
88976B/49, Derwent Publications Ltd., Lon-
don, GB&NUM;

(73) Proprietor: SUMITOMO HEAVY INDUSTRIES,
LTD
2-1 Ohtemachi 2-chome
Chiyoda-ku
Tokyo 100 (JP)

Proprietor: RIKAGAKU KENKYUSHO
2-1 Hirosawa
Wako-shi Saitama-ken (JP)

(72) Inventor: Endo, Isao
5-7-6, Honda
Kokubunji-shi Tokyo (JP)
Inventor: Nagamune, Teruyuki
2nd-chou-Manshio
404, 2-16-14, Chuo
Kamifukuoka-shi Saitama (JP)
Inventor: Tachikawa, Susumu
240-14, Nakamurahara
Odawara-shi Kanagawa (JP)
Inventor: Seike, Yasuhiro
1032-2, Maekawa
Odawara-shi Kanagawa (JP)
Inventor: Inaba, Hideki
Seimei-ryo
1-25-5, Iwase
Kamakura-shi Kanagawa (JP)
Inventor: Kurusu, Haruo
3681-2, Koshigaya
Koshigaya-shi Saitama (JP)

(74) Representative: **Bühling, Gerhard, Dipl.-Chem. et al**
**Patentanwaltsbüro**
**Tiedtke-Bühling-Kinne & Partner**
**Bavariaring 4**
**D-80336 München (DE)**

## Description

The present invention relates to a bioreactor which gives rise to high efficiency. Particularly it relates to a tubular bioreactor incorporating a reacting vessel and a separating membrane, in which reaction and separating operation can be carried out continuously in the same vessel.

The words "tubular bioreactor" herein is used to describe a reactor in which a flow containing biologically active catalyst ( hereinafter refering to as "biocatalyst" ) and reacting agent(s) can be circulated in a closed tubular circuit, with reacting together and the resulting material(s) can be discharged through a membrane mounted at least on a portion of the tubular wall in the form of tubing or forming a portion of tube wall.

In the prior art, the reaction using biocatalysts such as microorganisms and enzymes is difficult to be carried out in continuous form, and there was little success in commercialization of such continuous biocatalyzed process. There are many reasons; for example, it is difficult to adjust the concentration of microorganism in case of using microorganism, and it is difficult to recover and reuse costly enzyme in case of using enzyme.

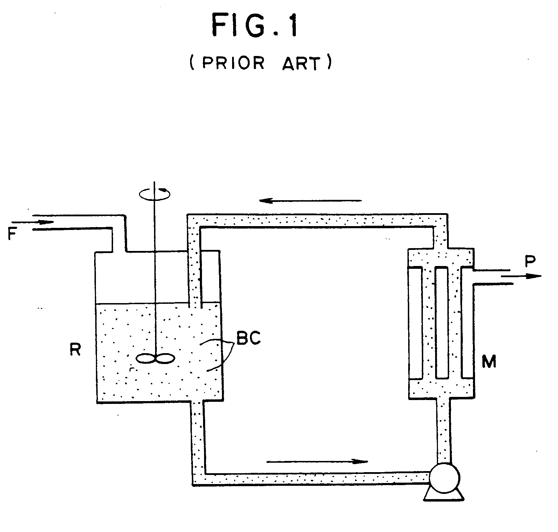

There have been proposed many approaches to carry out a continuous process for biocatalyzed reaction at a laboratory level, and one of them is as shown by FIG. 1 (the prior art), in which the reacting vessel and separating membrane are combined to form an apparatus which is schematically shown by FIG. 1. The biological reaction can be carried out continuously in the apparatus as shown in FIG. 1, wherein most of the reaction occurs in the reacting region R, and the separation of the resulting product from the mixture containing the biocatalyst is executed by passing through the porous membrane M. However, when the amounting volume other than the volume of reacting region R is raised to a higher ratio of the volume compared to the working volume, the role of the reacting vessel R is lowered in terms of place for reaction, and then, particularly in case of using a tubular separating membrane, the ratio of the volume of the other region than the reacting vessel to that of the reactor R is highly increased. Such bioreactor based on the combination of the conventional reactor and separating membrane is liable to be complicated in its structure, and then , there is much chance of being contaminated or polluted with harmful microorganism. Therefore, the prior art apparatus for continuous process is improper for commercial plant.

EP-A 195 094 describes a method for the fermantative production of organic solvents like acetone and butanol and a device, i.e. a fermenter used therefore. The continuously working fermenter is continuously fed with a substance like starch or sugar and water, each via an independent supply line. Furthermore, the fermenter is connected with a membrane unit via a connection line. In the membrane unit the fermenter substance flow is divided into a fermentation fluid, which flows through the membrane unit and is returned to the fermenter via another line, and a permeate flow representing the filtrate and being completely free of solids. Additionally, a small part of the fermenter substance flow is drawn off continuously via a seperate line.

US-A 3 969 190 describes a closed system in the form of a loop, wherein micro-organisms, cells bacteria, molds and the like can be cultivated. The loop system shows two filter membrane mass exchangers, wherein metabolites are removed via a metabolite accumulator; furthermore the apparatus according to this reference is provided with two dialysis membrane masse exchangers for replenishing nutrients.

The inventors have been investigating on research of efficient process for biocatalyzed reaction, and at last developed a novel apparatus for continuous process of biological reaction; that is, an tubular united bioreactor incorporating a biological activator containing reactor and a separating membrane, in which the liquid flow containing a biocatalyst and reacting agent(s) and product(s) is circulated while exerting biological reaction.

The words "tubular bioreactor" hereinafter is used to describe a reactor in which a flow containing biocatalysts such as enzyme and microorganism and reacting agent(s) can be circulated in a closed tubular circuit, while reacting together and the resulting material(s) can be discharged through a membrane mounted at least on a portion of the tubular wall in the form of tubing or forming a portion of tube wall.

Therefore, it is one object of the invention to provide a bioreactor having simplified structure, with less contamination and adapted to carry out continuous process of biological reaction.

It is a further object of the invention to provide a system for biocatalyzed reaction, in which less free surface of gas and liquid is enclosed within a simplified structure resulting in less trouble due to bubbling often found in fermentation reactor.

It is another object of the invention to provide a bioreactor with much flexibility, such as easy to scale up and down the facilities because of its simplicity and structure, easy to redesign the apparatus, which facilities can be compact and needs only small space.

These objects are achieved by the tubular bioreactor according to claim 1. The invention is advantageously developed by the measures mentioned in the subclaims.

FIG. 1 is a schematic view in section of the prior art bioreactor proposed for continuous process of biological reaction.

FIG. 2 is a sectional schematic representation of a tubular bioreactor of the present invention.

FIG. 3 is another sectional schematic representation of another embodiment of the tubular bioreactor of the present invention.

FIG. 4 is other sectional schematic representation of a tubular bioreactor with degassing means to resolve bubble or foaming problem, in accordance with the present invention.

## Detailed Description of Preferred Embodiments

The novel structure of a bioreactor in accordance with the present invention constitutes a tubular structure in form of closed loop, comprising tube(s), and tubular membrane element(s), in which a biological process can be continuously carried out to produce useful product(s) and the product(s) can be separated or discharged from the reaction flow or the reactor at the same time. Therefore, the inventive apparatus does not have special region such as the rector R in FIG. 1, and the biological reaction is carried out throughout in all portions of the tubular structure of the inventive apparatus.

The apparatus of the present invention is a bioreactor having a closed circuit structure, in which the reaction mixture containing a biocatalyst (enzyme or microorganism), a substrate(s) and a product(s) is circulated wherein the useful material or product is produced by a biological reaction, or in a certain case the useful microorganism is produced in a culture that is the liquid mixture circulated in the circuit structure. The closed tubular circuit structure of the apparatus of the present invention comprises porous tubular membrane module(s), through which the useful product can permeate so as to be separated. The membrane module is provided with the tube or part coaxially about the membrane module. The which tube or part of it is provided with an outlet for the product to be discharged on the wall of the tube coaxial about the membrane module.

Membrane modules to be used in the invention comprise a number of small porous membrane tubes bundled in tubular form, and the reaction mixture flows in the inner hollows of the said small porous membrane tubes. Preferably, the total sectional area of all inner hollows of the small porous membrane tubes equals substantially to the inner sectional area of the other connecting tube or pipe. The tubular member surrounding and enclosing the bundle of the small membrane tubes, and provided with an outlet for discharging the product is coaxial about the bundled tubular form of the membrane module. Further, the connecting structure between the tubular membrane module and the other hollow tube or pipe comprises a similar structure to that of heat exchanger in multiple pipe tubular form.

Further, the closed tubular circuit structure of the invention is provided with an inlet for feeding the substrate into the reactor. The inlet is provided on the wall of the tube constituting other than the membrane module, and connecting these parts constituting the circuit structure of the inventive apparatus. The closed circuit structure of the invention is provided with means for circulating the reaction mixture containing the substrate, the biocatalyst and the product and is further provided with the tube(s) which are connecting these parts constituting the above circuit structure.

Further, an outlet for draining the reaction mixture from the circuit reactor may be provided to pick up the useful microorganism contained in the reaction mixture. In addition, a degassing means can be provided in the closed circuit structure of the invention so as to control the gas or bubble amount in the mixture. Further, an oxygen inlet and a heat exchanger can be provided in the closed circuit structure of the inventive reactor.

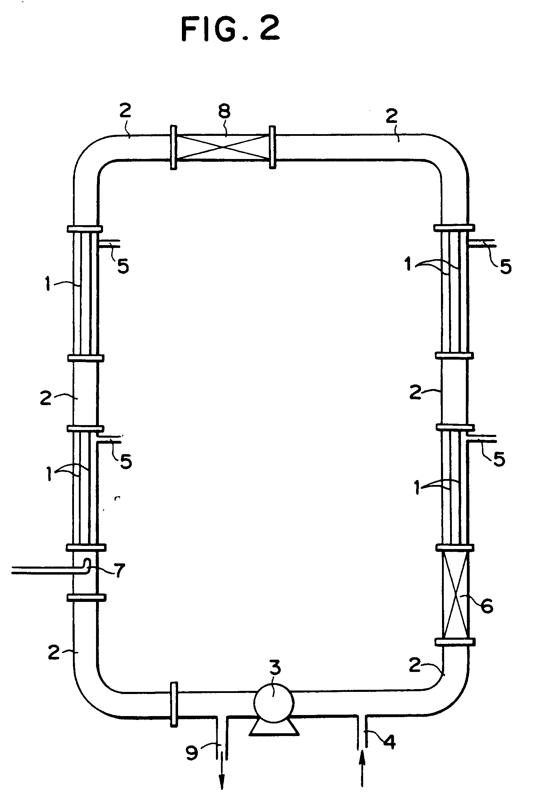

Referring now to the drawings for a more complete understanding of the present invention, a schematic illustration in section of a bioreactor system of the present invention is presented in FIG. 2.

The reaction mixture is circulated in the closed tubular circuit structure of the invention comprising ceramic or organic porous tubular membrane modules 1, through which biocatalyst cannot pass nor permeate; tubings 2 connecting the tubular membrane modules 1, with pump 3 for circulating the mixture liquid in the closed circuit as incorporated, and with a inlet 4 for feeding the starting materials.

While a pump is shown in the drawings, it may be any kind of means of generating the liquid circulating power to make the liquid circulate in the closed loop circuit. Further the closed circuit structure of the invention is provided with an inlet 4 for feeding the substrate, an outlet 5 for discharging the permeate which has passed through the tubular membrane module 1, and with a heat exchanger 6 which can adjust the temperature in the reactor within the predetermined temperature range adapted to the biological reaction. Further, numeral 7 is a feeder of oxygen or air when the biocatalyst needs oxygen in case of aerobic reaction. Further, a degassing means 8 is provided to reduce the amount of the excess gas in case of oxygen feeding. Further, the gas hold-up amount remaining in the reactor by generating metabolic gas can be maintained at the predetermined level. In general, hydrophobic porous membrane can be used for this degassing means. Further, outlet 9 may be provided to discharge the mixture of the substrate, the biocatalyst and the product, if desired.

Among the parts constituting the tubular circuit as shown in FIG. 2, the means for feeding oxygen and air 7, the degassing means 8, and the outlet 9 for drawing the product(s) can be omitted depending on the species of the biological reaction to be used.

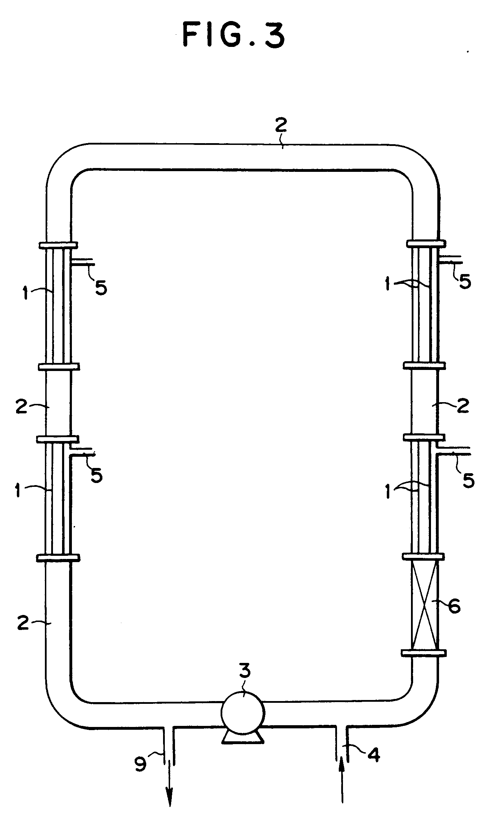

FIG. 3 illustrates a modified embodiment of the inventive tubular bioreactor wherein like reference characters designate like or corresponding parts or parts as shown in FIG. 2.

The position of each elements or components constituting the closed circuit structure is not restricted to those shown in the drawings including FIGS. 2, 3 and 4. The constitution of the closed loop circuit structure is only the requirement for the invention.

Further, the tubular membrane module 1 can be provided substituting partly in place of the part(s) of the tubing 2, thereby enhancing the separating efficiency of the product from the reaction mixture.

The dimensions of each parts or components constituting the closed circuit structure in the invention as shown in FIG. 2 can be determined in the following manner. As mentioned above, the part of the tube connecting each components can be substituted by a tubular membrane module. The minimum length of the tubular membrane module(s) in the enclosed structure of the inventive bioreactor can be calculated as follows:

Condition; membrane module is 150 tubes of inner pressure tubular membrane of 1200 mm in length $\times$ 10 mm in outer diameter $\times$ 6 mm in inner diameter, and then the membrane surface area is 3.4 m$^2$ with module inner diameter of 165 mm.

Where 4 modules are combined in series into the closed loop, the total membrane surface area $\Sigma S$ is;

$$\Sigma S = 13.6 m^2$$

When the permeation flux $Q_f$: $Q_f = 50$ $\ell$/hour$\cdot$m$^2$, then, the total amount of the permeating flow $\Sigma Q_f$ is;

$$\Sigma Q_f = 50 \times 13.6 = 680 \ \ell/hour$$

When the dilution rate at maximum is 2 hour$^{-1}$ (that means average retention time of 0.5 hours), the reaction volume $V_w$ of the tubular bioreactor is ;

$$V_w = \Sigma Q_f/2 = 340 \ \ell$$

The retentate volume of the module $V_d$ is;

$$V_d = 16.9 \ \ell$$

Accordingly, the volume V of the space other than the membrane module is;

$$V = V_w - V_d = 323.1 \ \ell$$

When the tube having the same inner diameter as that of the membrane module is used to connect the membrane module, the total length $L_p$ of the connecting tube is;

$$L_p = V/(\pi/4 \times 16.5^2) = 323.1 \times 10^3/(\pi/4 \times 16.5^2) = 1512 \ cm = 15.1 \ m$$

The total length $L_M$ of the membrane module is; $L_M = 1.2 \times 4 = 4.8$ m.

The ratio r of the length $L_M$ of the membrane module portion to the total length L of the tubular circuit is;

$$r = L_M /( L_p + L_M ) = L_M /L = 0.24$$

When the maximum dilution rate equals to 2 hour$^{-1}$, and the inner diameter of all tubular circuit is equal, the minimum ratio of the membrane module length to the total length of the closed circuit is almost 24%. If the inner diameters of all tubular circuit are the same, the ratio of the membrane module length to the total length of the closed circui is 24 % to 100 %. However, when the inner diameter of the membrane module is different from that of the tube(s) connecting the module and the other components, the ratio of the membrane module length to the total length of the circuit becomes different.

The function of the inventive tubular bioreactor is exerted as follows, so as to recover continuously the useful product from the reaction mixture produced by the biocatalyzed reaction.

In case of anaerobic fermentation, or enzyme reaction without generating metabolic gas, the bioreactor as shown FIG. 3 is used to carry out the biological reaction.

The sterilized or pasteurized substrate(s) and the biocatalyst were put in the inventive tubular bioreactor and then the mixture containing the biocatalyst and the substrate(s) was circulated under the appropriate condition to start the biological reaction. After starting the process, the substrate(s) is continuously fed from the inlet 4. The materials are circulated with the biocatalyst in the tubular circuit of the reactor, while being converted into the useful product by the biocatalyst. In the tubular bioreactor, a membrane1 is provided in place of the part(s) of the tubular structure in which the reaction mixture is circulated. The liquid rich in the product and not containing the biocatalyst passes through the membrane 1, and the amount of the liquid equal t biocatalyst that of the feed is discharged from the outlet 5. The biocatalyst is maintained in the tubular circuit of the inventive bioreactor because the biocatalyst cannot pass through the membrane module 1. Therefore, the material(s) can be continuously processed. In the other words, the biocatalyzed reaction and the separation of its product can be effected simultaneously and continuously. A heat exchanger can be provided or inserted in the circuit, if it is necessary, so as to adjust the temperature of the mixture in the reactor to the predetermined level. If the microorganism produced by the biological reaction in the reactor is useful and an object of the process, the outlet 9 for discharging the liquid containing the microorganism is provided at appropriate position of the tubular structure, so as to recover the mixture containing the useful microorganism.

If the anaerobic fermentation accompanies generation of metabolic gas, the tubular reactor as shown in FIG. 2 is used to have a degassing means in the way of the tubular structure, so as to remove generated metabolic gas from the reactor.

When the biological reaction is aerobic fermentation, the tubular structure as shown in FIG. 2 is used. Oxygen or air is fed from an gas inlet 7 to the reaction mixture, and the excess gas remaining in the tubular structure, and the metabolic gas as generated can be removed so as to maintain the gas hold-up amount in the reactor below the predetermined level.

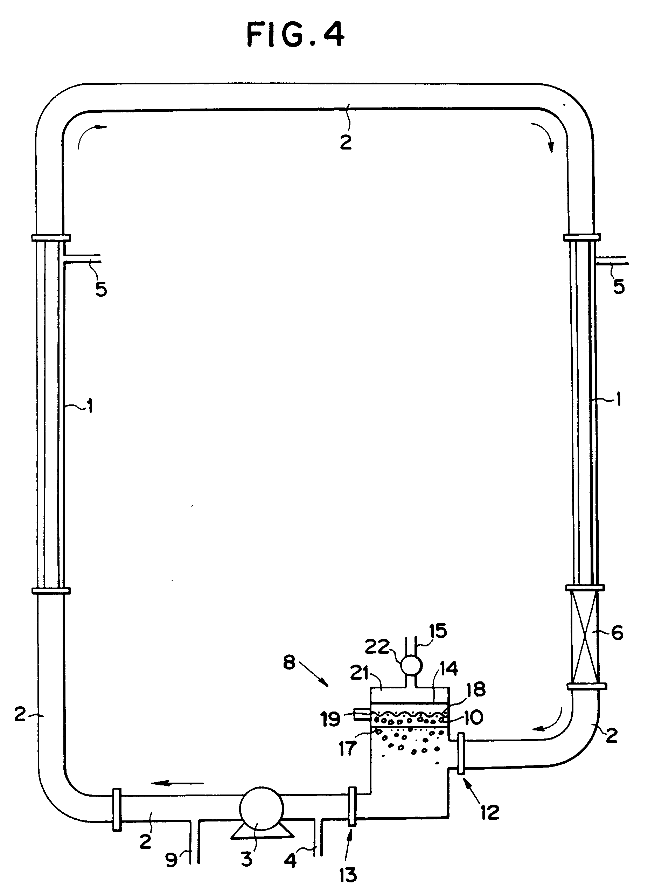

The other modified embodiment of the invention seen in FIG. 4 illustrates another way of removing efficiently the metabolic gas and bubble generated in the tubular circuit structure of the invention. Generally speaking, bubble forming or foaming may be problem in the bioreactor. The used microorganisms adhered on the surface of foam. As the result of this phenomena, the concentration of the active microorganism in the reaction mixture is decreased. Then, it will give rise to the lowering of productivity, and when foaming is much, the reaction mixture would be disposed or discharged together with foams so as to loose the useful product(s).

In order to avoid such foaming problem, the tubular structure of the inventive bioreactor as seen in FIG. 4 can be used.

This modified embodiment of the invention has a degassing means for removing excess gas or foams in the reactor, which has the following structure.

A gas retention hollow space is provided directly above the surface of the reaction liquid restrained in the portion of the circuit structure, so as to remove metabolic gas or excess air held in the mixture. A hydrophobic membrane is further provided at the position of the upper level of the gas retention hollow space and an exhaust outlet is provided above the hydrophobic membrane part as to drain the exhaust gas. Further, it is preferable to provide heatable metal wire gauze part between said hydrophobic membrane part and said surface of the liquid in the hollow of the gas retention space.

The hydrophobic membrane used for the invention is preferably heat resisting so that it can be steam pasteurized, for example, can be heated stably at least to 120 °C for steam pasteurization. Gas can permeate through the hydrophobic membrane, but liquid cannot permeate through. Further, the strength is chosen sufficiently high to prevent breaking even if the microorganism adheres or in case of sealing up the media. In addition the membrane has fine pores of smaller than 0.2 micrometer enough to prevent the microorganism from permeating through the membrane.

As to materials to be used for the hydrophobic membrane, a variety of experiments were carried out to find that polypropylenes resin and polyamides resin are weak to repeated heating for pasteurization, and porous fluorides resin is preferable to be enough resistant to the repeated heating.

Further, metal wire gauze that can be heated to the appropriate temperature may be preferably provided at the position between the hydrophobic membrane and the surface of the liquid, so as to facilitate breaking bubbles or foams. When the bubble is contacted with the heated metal wire gauze, it will be easily broken. The heating means may be an electric current to pass the wire gauze, and the heating to the temperature of about 80 °C is enough to effectively break the bubbles or foams.

FIG. 4 illustrates a modified embodiment of the invention in which a degassing means 8 is provided so as to effect efficient removal of bubbles or foams from the reaction mixture.

In this embodiment, the reaction mixture flow is led into the degassing space 8 through an inlet opening 12. The degassing means 8 has a gas retention hollow space 21 at the upper portion thereof, in which space 21 a hydrophobic membrane 14 of porous poly fluoride resin is provided. The gas in the reaction flow will float up in the degassing means 8 having the section area larger than that of the connecting tubular pipe 2 because the flow will not move so rapidly and the pressure is reduced at the degassing means 8. The floating gas will become or generate bubbles or foams 10 which will reach to a hydrophobic membrane 14 of gas- permeating but liquid not -permeating properties, and compressed between the following foam 10 and the membrane 14 to be broken. Gas generated by breaking the foams will pass the hydrophobic membrane 14, and is discharged from the tubular bioreactor through an exhaust outlet 15.

On the other hand, the mixture liquid remaining or as recovered at the degassing means 8 will flow through the bubbles as retained in the space 21 and return into the main mixture flow.

The exhaust outlet 15 is provided with a pressure regulator 22 so as to regulate the pressure of the gas in the degassing means 8 thereby attaining the appropriate condition of degassing.

Further, a metal wire gauze 18 is preferably provided at the position between the hydrophobic membrane 14 and the surface 17 of the reaction liquid so as to facilitate breaking bubbles in a gas retention hollow space 21. The metal wire gauze 18 may have a heater 19, and can be heated at an appropriate temperature. Then, when bubble touches the heated metal wire gauze 18, it can be easily broken to form gas because there will occur difference in surface tension of the touched surface of the foam and the untouched surface thereof.

The circuit structure of this modified embodiment of the inventive tubular bioreactor comprises a separation membrane 1, tubular connecting pipe 2, pump 3, an feed inlet 4, an outlet 5 for permeate, heat exchanger 6 and a drain outlet 9 for the reaction mixture, like as shown in FIG. 2.

In the above description, the invention is explained in reference to the bioreactor using microorganism or enzyme. Embodied usage of the inventive tubular bioreactor may include a lactic acid fermentation by utilizing glucose with microorganism such as Lactobacillus. Other usage may include alcoholic fermentation using yeast organism. In the latter case, molasses can be used for a substrate so as to enable compact and uncostly reactor for biocatalyzed reaction.

The tubular bioreactor of the present invention shows a simple structure so that it would be less polluted with harmful organisms to enable continuous operation. The continuous operation needs continuous feeding of the substrate(s), which can be attained by continuous removal of microorganism of the substrate using a membrane.

The closed circuit structure of the invention has little free surface of gas and liquid, and then there is less chance of gas putting into the liquid mixture, which enables resolution of bubble or foam problem during the process of fermentation. The basic structure of the apparatus is a circulating closed piping, and therefore, the apparatus of the invention can be easily scaled up or down, and further, can be compacted because of its simple structure and small space of its facilities.

In the modified embodiment as shown in FIG. 4, there is no cost for running a degassing means because of no need of driving' force for degassing. In addition, there is no need of space for generated foam, and therefore, the working volume of the inventive bioreactor would be almost 100 percent.

Further, while the conventional reactor requires filter to avoid pollution with harmful organism in an exhaust, the hydrophobic membrane in a degassing means functions as a filter so that there is no need of special filter.

## Claims

1. A tubular bioreactor in which useful product(s) is (are) produced in a liquid mixture flow flowing therein containing fluidized biocatalysts and substrates, said tubular bioreactor having the structure of a tubular circuit in a closed loop comprising:
   - means (3) for continuously circulating the mixture flow containing said substrates, biocatalysts and products in the tubular circuit structure,
   - an inlet (4) for feeding substrates to the circuit said inlet being mounted on the wall of
   - tubular hollow members (2) in which the liquid mixture continuously flows and circulates and which form the closed loop of the tubular circuit and
   - porous tubular membrane modules (1), which
     - show outlets (5) for continuously discharging the products from the tubular circuit structure and
     - are provided with said outlets (5) on the outer surface and
     - have a number of small porous hollow membrane tubes in which the liquid mixture continuously flows and wherethrough the product(s) can pass or permeate so as to discharge the product(s) from the circuit, said tubular membrane modules constituting at least one portion of the tubular circuit and being coaxial about and spaced from the tubular hollow members (2)

2. A bioreactor according to claim 1 wherein means (9) for withdrawing the mixture flow containing the substrates, the biocatalyst and the products from the circuit are provided in said closed tubular circuit structure downstream of said means (3) for circulating the flow.

3. A bioreactor according to claim 1 or 2, wherein a degassing means (8) is mounted in the closed circuit structure.

4. A bioreactor according to claim 3, wherein a means (7) for feeding oxygen or air to the liquid mixture flow is mounted in the closed circuit structure.

5. A bioreactor according to any of claims 1 to 4, wherein a heat exchanger (6) is provided in said closed circuit structure.

6. A bioreactor according to claim 3, wherein said degassing means (8) comprises:
   - a gas retention hollow space (21) forming gas phase portion on and directly above the upper surface of the mixture flow restrained in the degassing means (8);
   - a hydrophobic membrane (14) having the ability of gas permeating and liquid not-permeating, provided at the level of upper portion in said gas retention space (21); and
   - a gas exhaust outlet (15) for discharging gas from the bioreactor, provided above said hydrophobic membrane in the gas retention space.

7. A bioreactor according to claim 6, wherein further a metal wire gauze (18) which can be heated is further provided between said hydrophobic membrane and said surface of the restrained liquid in the degassing means.

**Patentansprüche**

1. Röhrenförmiger Bioreaktor, in dem mindestens ein nützliches Produkt in einer Flüssigkeitsmischungsströmung hergestellt wird, die in dem Reaktor strömt und die fluidisierte Biokatalysatoren und Substrate aufweist, wobei der röhrenförmige Bioreaktor die Struktur eines röhrenförmigen Kreislaufs mit einer geschlossenen Schleife hat und:
   Mittel (3) zur kontinuierlichen Zirkulation der Mischungsströmung, welche die Substrate, die Biokatalysatoren und die Produkte in der röhrenförmigen Kreislaufstruktur enthält,
   einen Einlaß (4) zur Zuführung von Substraten zu dem Kreislauf, wobei der Einlaß an der Wand von
   röhrenförmigen hohlen Elementen (2) angeordnet ist, in denen die Flüssigkeitsmischung kontinuierlich strömt und welche die geschlossene Schleife des röhrenförmigen Kreislaufs bilden, und
   poröse röhrenförmige Membranmodule (1) aufweist, die Auslässe (5) für eine kontinuierliche Abgabe der Produkte aus der röhrenförmigen Kreislaufstruktur haben, wobei
   die Auslässe (5) an der äußeren Fläche der Module vorgesehen sind, und
   eine Zahl von kleinen porösen hohlen Membranröhren aufweisen, in denen die Flüssigkeitsmischung kontinuierlich strömt und durch die mindestens ein Produkt hindurchtreten oder sie durchdringen kann, um das mindestens eine Produkt aus dem Kreislauf zu entfernen, wobei
   die röhrenförmigen Membranmodule wenigstens einen Abschnitt des röhrenförmigen Kreislaufs darstellen und koaxial zu und beabstandet von den röhrenförmigen hohlen Elementen (2) vorliegen.

2. Bioreaktor nach Anspruch 1, wobei Mittel (9) zum Entfernen der Mischungsströmung aus dem Kreislauf, wobei die Mischungsströmung die Substrate, den Biokatalysator und die Produkte enthält, in der geschlossenen röhrenförmigen Kreislaufstruktur stromab von den Mitteln (3) zur Zirkulation der Strömung vorgesehen sind.

3. Bioreaktor nach Anspruch 1 oder 2, wobei Entgasungsmittel (8) in der geschlossenen Kreislaufstruktur angeordnet sind.

4. Bioreaktor nach Anspruch 3, wobei Mittel (7) zur Zuführung von Sauerstoff oder Luft zu der Flüssigkeitsmischungsströmung in der geschlossenen Kreislaufstruktur angeordnet sind.

5. Bioreaktor nach einem der Ansprüche 1 bis 4, wobei ein Wärmeaustauscher (6) in der geschlossenen Kreislaufstruktur vorgesehen ist.

6. Bioreaktor nach Anspruch 3, wobei die Entgasungsmittel (8):
   einen Gasretentionshohlraum (21), der einen Gasphasenabschnitt an und direkt über der oberen Fläche der Mischungsströmung, die in den Entgasungsmitteln (8) zurückgehalten wird, bildet;
   eine hydrophobe Membran (14), durch die Gas, aber keine Flüssigkeit hindurchtreten kann und die auf der Ebene eines oberen Abschnitts in dem Gasretentionsraum (21) vorgesehen ist; und
   einen Gasausströmauslaß (15) zur Abgabe von Gas aus dem Bioreaktor aufweisen, wobei der Auslaß oberhalb der hydrophoben Membran in dem Gasretentionsraum angeordnet ist.

7. Bioreaktor nach Anspruch 6, wobei ferner ein erhitzbares Metalldrahtgewebe (18) zwischen der hydrophoben Membran und der Oberfläche der zurückgehaltenen Flüssigkeit in dem Entgasungsmittel angeordnet ist.

## Revendications

1. Un bioréacteur tubulaire dans lequel une ou plusieurs substances sont produites dans un flux d'un mélange liquide qui s'y écoule, et qui contient des biocatalyseurs et des substrats, ledit bioréacteur tubulaire présentant la structure d'un circuit tubulaire en boucle fermée comprenant:
   - un moyen pour faire circuler en continu le flux de mélange contenant lesdits substrats, les biocatalyseurs et les produits dans la structure de circuit tubulaire,
   - une entrée (4) pour amener des substrats au circuit, ladite entrée étant montée sur la paroi d'organes tubulaires creux,
   - des organes tubulaires creux (2), dans lesquels le mélange liquide s'écoule et circule en continu et qui forment la boucle fermée du circuit tubulaire et
   - des modules à membrane (1) tubulaires poreux, qui :
     - présentent des sorties (5) pour évacuer en continu les produits depuis la structure de circuit tubulaire,
     - sont pourvus desdites sorties (5) sur la surface extérieure et
     - présentent un certain nombre de tubes à membrane creux, poreux, dans lesquels le mélange liquide s'écoule en continu et à travers lesquels le ou les produits peuvent passer ou s'infiltrer, de manière à évacuer le ou les produits hors du circuit, lesdits modules tubulaires à membrane coant au moins une partie du circuit tubulaire, étant coaxiaux aux organes creux tubulaires (2) et étant espacés de ces organes.

2. Un bioréacteur selon la revendication 1, dans lequel un moyen (9) pour extraire le flux de mélange contenant les substrats, le biocatalyseur et les produits, hors du circuit, est prévu dans ladite structure tubulaire fermée, en aval dudit moyen (3) servant à faire circuler le flux.

3. Un bioréacteur selon la revendication 1 ou 2, dans lequel un moyen de dégazage (8) est monté dans la structure de circuit fermé.

4. Un bioréacteur selon la revendication 3, dans lequel un moyen (7), servant à l'apport d'oxygène ou de l'air au flux de mélange liquide, est monté dans la structure de circuit fermé.

5. Un bioréacteur selon l'une quelconque des revendications 1 à 4, dans lequel un échangeur de chaleur (6) est prévu dans ladite structure de circuit fermé.

6. Un bioréacteur selon la revendication 3, dans lequel ledit moyen de dégazage (8) comprend :
   - un espace creux de rétention de gaz (21), formant une partie de phase gazeuse sur la surface supérieure du flux de mélange au-dessus d'elle, en étant maintenu de manière forcée dans le moyen de dégazage (8);
   - une membrane hydrophobe (14) présentant la caractéristique de perméabilité aux gaz et d'imperméabilité aux liquides, prévue au niveau de la partie supérieure dans ledit espace de rétention de gaz (21);
   - une sortie d'échappement de gaz (15) pour évacuer le gaz depuis le bioréacteur, prévue au-dessus de ladite membrane hydrophobe, dans l'espace de rétention de gaz.

7. Un bioréacteur selon la revendication 6, dans lequel une gaze métallique (18), qui peut être chauffée, est en outre prévue entre ladite membrane hydrophobe et ladite surface du liquide maintenu dans le moyen de dégazage.

# FIG.1

## (PRIOR ART)

EP 0 295 567 B1

# FIG. 2

11

# FIG.3

EP 0 295 567 B1

# FIG.4

13